# EUROPEAN PATENT APPLICATION

(11) **EP 4 385 518 A1**
(43) Date of publication of application: **19.06.2024**
(21) Application number: 22214262.2
(22) Date of filing: 16.12.2022
(51) Int. Cl.: A61K 38/44, A61K 47/51, A61K 47/54, A61K 47/68, A61P 35/00, C07F 5/02

(54) **KIT FOR COMBINATION THERAPY OF TUMOR DISEASES AND/OR INFECTIONS**

(71) Applicant: Julius-Maximilians-Universität Würzburg, 97070 Würzburg (DE)
(72) Inventor: The designation of the inventor has not yet been filed
(74) Representative: Engelhard, Markus

(57) **Abstract**

The present invention relates to a kit of active pharmaceutical ingredients comprising (1) a fusion component comprising an agent, which increases the level of reactive oxygen species (ROS), such as Aplysia punctata ink toxin (APIT), and a targeting sequence, and (2) a prodrug or a proreporter comprising phenylboronic acid derivatives. The present invention relates to said kit of pharmaceutical compositions for use in the diagnosis, prevention and/or treatment of proliferative diseases, viral and/or non-viral infections. The present invention further relates to a method of diagnosis, prevention and/or treatment of a proliferative disease, a viral infection and/or a non-viral infection.

## Description

The present invention relates to a kit of active pharmaceutical ingredients comprising (1) a fusion component comprising an agent, which increases the level of reactive oxygen species (ROS), such as Aplysia punctata ink toxin (APIT), and a targeting sequence, and (2) a prodrug or a proreporter comprising phenylboronic acid derivatives. The present invention relates to said kit of pharmaceutical compositions for use in the diagnosis, prevention and/or treatment of proliferative diseases, viral and/or non-viral infections. The present invention further relates to a method of diagnosis, prevention and/or treatment of a proliferative disease, a viral infection and/or a non-viral infection.

### BACKGROUND OF THE INVENTION

In the treatment of almost all diseases, such as cancer and chronic inflammation, the aim is to achieve therapeutic success with as few side effects as possible. Therefore, a combination of reliable diagnostic tools and specific medication is the basis of any therapy. In the last decades, so-called "smart systems" have been investigated in the fields of biomedicine and nanoscience, where the release of the drug is controlled by different triggers, in particular pH, temperature, redox reactions, (bio)chemical reactions and so on ("prodrug" strategy). In addition, many studies have been conducted that address targeted, site-specific delivery ("targeting") of drugs. Almost all these systems have specific limitations, including safety, efficacy, and selectivity, so new approaches will need to be developed in the future.

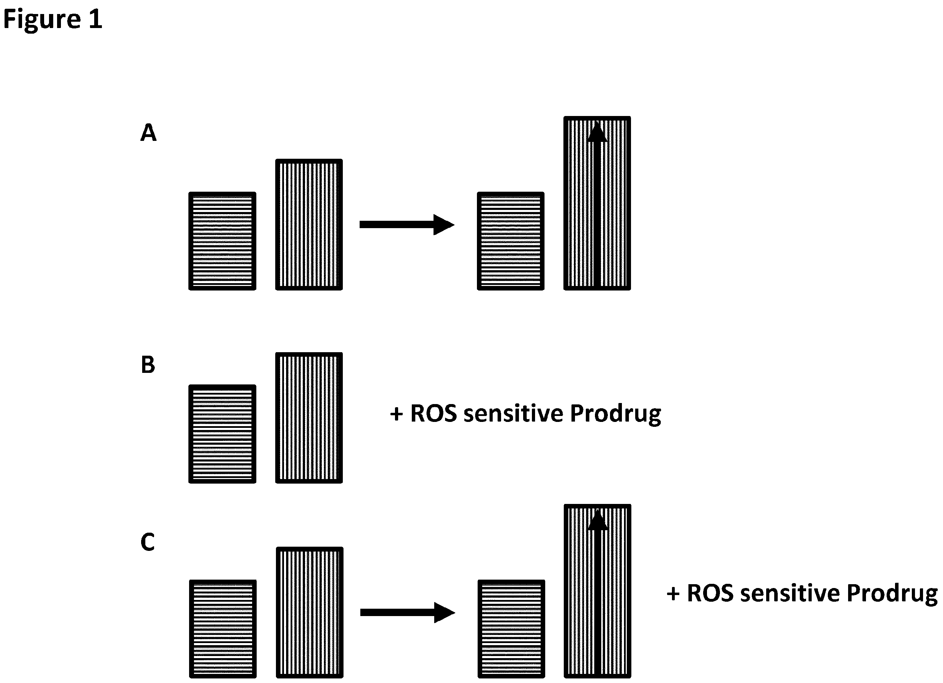

In recent years, the role of reactive oxygen species (ROS) has been highlighted, acting as regulators of various cellular functions (Sies and Jones, 2020). Increases in ROS are observed in various pathological conditions such as tumor promotion and progression (Perillo *et al*.*,* 2020), Huntington's disease (Johri and Beal, 2012), Alzheimer's disease (Mattson, 2004), and Parkinson's disease (Exner *et al.,* 2012). Moreover, it is known that highly elevated levels of ROS can trigger programmed cell death (Reczek and Chandel, 2017). As a result, cancer treatment strategies based on modulation of ROS levels have been described (Perillo *et al*.*,* 2020) (as shown in Figure 1 A).

In addition, the intrinsic ROS gradient between healthy and diseased tissues can be used to control drug release (Peiró Cadahía *et al.,* 2019) (see Figure 1 B). However, these strategies are critical because the ROS gradient is often not significantly mature, leading to uncontrolled hydrolysis and release of the drugs, thus providing insufficient selectivity.

There is a need in the art for improved means and methods for the treatment of cancer which provide improved selectivity and/or effectivity.

### SUMMARY OF THE INVENTION

According to the present invention this object is solved by a kit of active pharmaceutical ingredients comprising
(1) a fusion component comprising an agent, which increases the level of reactive oxygen species (ROS), and a targeting sequence, and
(2) a prodrug or proreporter comprising a compound of formula I or formula II 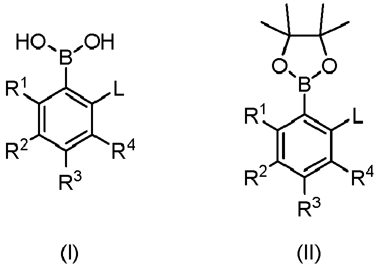 wherein
   **R¹** to **R⁴** are each independently selected from H, O**R⁵**, NH**R⁵**, NHC(O)**R⁵**, S**R⁵**, halogen, C₁-C₆ alkyl, C₁-C₆ alkoxy, aryl, and C₁-C₂₀ alkyloxycarbonyl,
   **R⁵** is selected from H, C₁-C₆ alkyl, C₁-C₆ alkoxy, and C₁-C₆ alkyl or C₁-C₆ alkoxy substituted with a reactive group for attachment of a polymer, peptide or protein,
   **L** is selected from -(CH₂)₂-C(O)O-**X** and -CH=CH-C(O)O-**X,** and
   **X** is a drug or a reporter,
   or a pharmaceutically acceptable salt thereof.

According to the present invention this object is solved by kit of active pharmaceutical ingredients of the present invention for use in the diagnosis, prevention and/or treatment of proliferative diseases, viral infections and/or non-viral infections.

According to the present invention this object is solved by a method for the diagnosis, prevention and/or treatment of a proliferative disease, a viral infection and/or a non-viral infection, comprising the administration of therapeutically effective amounts of the kit of active pharmaceutical ingredients of the present invention to a subject in need thereof.

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only and is not intended to limit the scope of the present invention which will be limited only by the appended claims. Unless defined otherwise, all technical and scientific terms used herein have the same meanings as commonly understood by one of ordinary skill in the art. For the purpose of the present invention, all references cited herein are incorporated by reference in their entireties.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "0.5 to 10" should be interpreted to include not only the explicitly recited values of 0.5 to 10, but also include individual values and sub-ranges within the indicated range. Thus, included in this numerical range are individual values such as 0.5, 1, 1.5, 2, 2.5, 3, 4, 5 .... 8, 8.5, 9, 9.5, 10 and sub-ranges such as from 2 to 8, 4 to 5, etc. This same principle applies to ranges reciting only one numerical value, such as "less than 1 mm". Furthermore, such an interpretation should apply regardless of the breadth of the range or the characteristics being described.

### Kits of active pharmaceutical ingredients

As outlined above, the present invention provides a kit of active pharmaceutical ingredients.

Said kit of active pharmaceutical ingredients comprises
(1) a fusion component comprising an agent, which increases the level of reactive oxygen species (ROS), and a targeting sequence, and
(2) a prodrug or proreporter comprising a compound of formula I or formula II 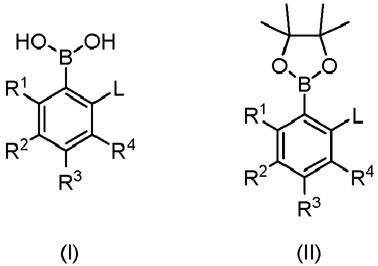 wherein
   **R¹ to R⁴** are each independently selected from H, O**R⁵**, NH**R⁵**, NHC(O)**R⁵**, S**R⁵**, halogen, C₁-C₆ alkyl, C₁-C₆ alkoxy, aryl, and C₁-C₂₀ alkyloxycarbonyl,
   **R⁵** is selected from H, C₁-C₆ alkyl, C₁-C₆ alkoxy, and C₁-C₆ alkyl or C₁-C₆ alkoxy substituted with a reactive group for attachment of a polymer, peptide or protein,
   **L** is selected from -(CH₂)₂-C(O)O-**X** and -CH=CH-C(O)O-**X**, and
   **X** is a drug or a reporter,
   or a pharmaceutically acceptable salt thereof.

Active ingredients are the substances in drugs that are responsible for the health effects/benefits experienced by the subjects. The active ingredient in a pharmaceutical drug is called an active pharmaceutical ingredient (API).

The kit of the present invention can further comprise pharmaceutically acceptable expedient(s) and/or carrier.

Said active pharmaceutical ingredients (1) and (2) are each provided as a pharmaceutical composition, i.e. as a pharmaceutical composition in itself.

They can further comprise pharmaceutically acceptable expedient(s) and/or carrier.

The kit of active pharmaceutical ingredients of the present invention can also be considered as a kit of parts, wherein the parts are the active pharmaceutical ingredients or their respective pharmaceutical composition.

The kit of the present invention can also be considered as a collection of the two active pharmaceutical ingredients (1) and (2).

### Active pharmaceutical ingredient (1) - fusion component

The active pharmaceutical ingredient (1) of the kit of the present invention is a fusion component comprising
an *agent, which increases the level of reactive oxygen species (ROS),* and
a *targeting sequence.*

The active pharmaceutical ingredient (1) has the task of selectively generating oxidative stress and the associated increase in ROS molecules at the site of action.

An agent, which increases the level of reactive oxygen species (ROS), can be selected from: beta-lapachone (ARQ 501), NOV-002 (a mimetic of glutathione disulfide), gamitrinib, bortezomib, celecoxib, imatinib, erlotinib, vermurafenib, rituximab, procarbazine, doxorubicin, arsenic trioxide, 2-methoxyestradiol, N-(4-hydroxyphenyl retinamide), platin agents, elesclomol (STA-4783), sanguinarine, rapamycin, D-amphetamine, and ferrocene und its derivates.

Preferably, the agent, which increases the level of reactive oxygen species (ROS), is a protein, more preferably an enzyme.

Examples for enzymes, which increase the level of reactive oxygen species (ROS), are glucose oxidases, amino acid oxidases, format oxidases, NADH oxidases, NADPH oxidases and nucleoside oxidases.

In one embodiment, the enzyme which increases the level of reactive oxygen species (ROS), is an L-amino acid oxidase (LAAO, EC 1.4.3.2). LAAO are highly specific towards different L-amino acids. LAAO are found in various species and show diverse physiological roles. LAAO are found primarily in various animal fluids, including serum, venoms, ink, mucus and mammary milk and tissue extract. So far, snake venom LAAO (SV-LAAO) are the most thoroughly investigated amino acid oxidases. function and bioactivity of LAAO include antibacterial and antiparasitic properties as a main defense of the organism or part of the immune defense. Said defense mechanism of LAAO can be primarily traced back to the generated H₂O₂.

In a preferred embodiment, the active pharmaceutical ingredient (1) of the kit of the present invention is a fusion protein comprising
*Aplysia punctata ink toxin (APIT),* and
a *targeting sequence.*

The *Aplysiapunctata ink toxin* (*APIT*) comprises or consists of the amino acid sequence of SEQ ID NO. 1.

APIT is a FAD-binding L-amino acid oxidase (Butzke *et al.,* 2005).

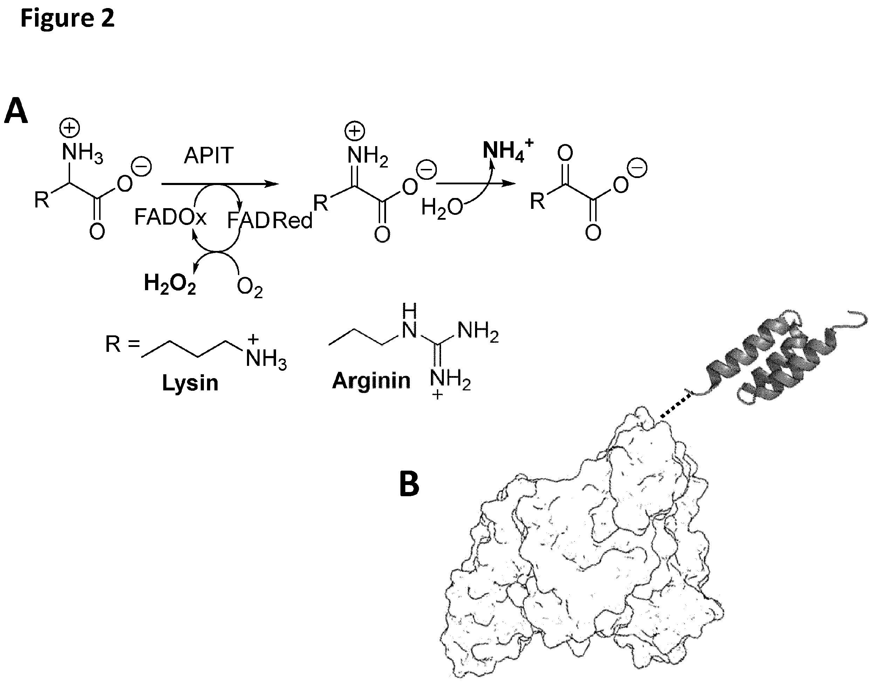

APIT has an antitumor effect which is based on the selective degradation of the essential L-amino acids L-lysine and L-arginine. This redox reaction produces as reactive byproducts hydrogen peroxide (H₂O₂) and NH₄⁺, which are also detrimental to the tumor. See e.g. Figure 2A.

According to the present invention, the protein, which increases the level of reactive oxygen species (ROS), such as APIT, also serves to increase the level or concentration of reactive oxygen species (ROS), such as H₂O₂, at the site of action, namely at the tumor site.

SEQ ID NO 1: amino acid number: 518, molecular weight: 58502.56

1 10 20 30 40 50 60
MDGVSRNRRQC NREVCGSTYD VAVVGAGPGG ANSAYMLRDS GLDIAVFEYS DRVGGRLFTY

70 80 90 100 110 120
QLPNTPDVNL EIGGMRFIEG AMHRLWRVIS ELGLTPKVFK EGFGKEGRQR FYLRGQSLTK

130 140 150 160 170 180
KQVKSGDVPY DLSPEEKENQ GNLVEYYLEK LTGLQLNGEP LKREVALKLT VPDGRFLYDL

190 200 210 220 230 240
SFDEAMDLVA SPEGKEFTRD THVFTGEVTL DASAVSLFDD HLGEDYYGSE IYTLKEGLSS

250 260 270 280 290 300
VPQGLLQAFL DAADSNEFYP NSHLKALRRK TNGQYVLYFE PTTSKDGQTT INYLEPLQVV

310 320 330 340 350 360
CAQRVILAMP VYALNQLDWN QLRNDRATQA YAAVRPIPAS KVFMTFDQPW WLENERKSWV

370 380 390 400 410 420
TKSDALFSQM YDWQKSEASG DYILIASYAD GLKAQYLREL KNQGEDIPGS DPGYNQVTEP

430 440 450 460 470 480
LKDTILDHLT EAYGVERDSI PEPVTAASQF WTDYPFGCGW ITWRAGFHFD DVISTMRRPS

490 500 510
LKDEVYVVGA DYSWGLISSW IEGALETSEN VINDYFL

The *targeting sequence* preferably targets cell types or tissues of interest, in particular cancer cells. The *targeting sequence* can also target viruses, bacterial cells or cells of other microorganisms.

In one embodiment, the targeting sequence targets cancer cells, such as human epidermal growth factor receptor (HER2)-overexpressing cells.

In other embodiments, the targeting sequence targets human immunodeficiency virus (HIV), hepatitis B virus (HBV) or/and hepatitis C virus (HCV).

The targeting sequence can be an antibody or an antibody fragment, a targeting peptide or peptide-like affibody, a nucleic acid or small targeting molecule.

Preferably, the active pharmaceutical ingredient (1) is a fusion protein, wherein the targeting sequence is preferably fused N-terminally to APIT.

In one embodiment, the active pharmaceutical ingredient (1) is a conjugate. Here, conjugation methods which are known in the art can be used to conjugate the targeting sequence to e.g. APIT. For example, conjugation via N-terminal modification using Schiff base reaction + reductive amination. For example, conjugation via modification of amino acids, such as lysine, using active esters preferably NHS-esters.

In one preferred embodiment, the targeting sequence is a variant of an antibody, antibody fragment, targeting peptide, peptide-like affibody or small targeting molecule, wherein the lysine residues are deleted or substituted, preferably with arginine and/or alanine.
Said elimination of lysine residues by deletion or substitution preferably has a thermodynamically stabilizing effect. Said elimination of lysine residues can also have an effect on binding to a respective target receptor, such as it can be less interfering with a receptor binding site.

For example, in an embodiment where the targeting sequence targets human epidermal growth factor receptor (HER2)-overexpressing cells, said elimination of lysine residues results in less interfering with the Her2/neu receptor binding site.

In a preferred embodiment, the targeting sequence is zHER2:342.
zHER2:342 comprises or consists of the amino acid sequence of SEQ ID NO. 2.

VDNKFNKEMR NAYWEIALLP NLNNQQKRAF IRSLYDDPSQ SANLLAEAKK
LNDAQAPKGG SGGS

In one embodiment, the targeting sequence is a zHER2:342 variant wherein the lysine residues are deleted or substituted, preferably with arginine and/or alanine. In one embodiment, the targeting sequence is K4R/K7A/K27R/K49R/K50A/K58A zHER2:342.

K4R/K7A/K27R/K49R/K50A/K58A zHER2:342 comprises or consists of the amino acid sequence of SEQ ID NO. 3.

SEQ ID NO. 3:
VDNRFNAEMR NAYWEIALLP NLNNQQRRAF IRSLYDDPSQ SANLLAEARA
LNDAQAPAGG SGGS

Such zHER2:342 variant wherein the lysine residues are deleted or substituted is highly suitable to be further modified, such as by PEGylation.

In one embodiment, the fusion protein is zHER2:342-APIT.
zHER2:342-APIT comprises or consists of the amino acid sequence of SEQ ID NO. 4:

SEQ ID NO 4:                amino acid number: 583, molecular weight: 65723.67 g/mol

1         10         20         30         40         50         60
MVDNRFNAEMR NAYWEIALLP NLNNQQRRAF IRSLYDDPSQ SANLLAEARA LNDAQAPAGG

70         80         90         100         110         120

GSGGGDGGGS MDGVSRNRRQ CNREVCGSTY DVAVVGAGPG GANSAYMLRD SGLDIAVFEY

130 140 150 160 170 180
SDRVGGRLFT YQLPNTPDVN LEIGGMRFIE GAMHRLWRVI SELGLTPKVF KEGFGKEGRQ

190 200 210 220 230 240
RFYLRGQSLT KKQVKSGDVP YDLSPEEKEN QGNLVEYYLE KLTGLQLNGE PLKREVALKL

250 260 270 280 290 300
TVPDGRFLYD LSFDEAMDLV ASPEGKEFTR DTHVFTGEVT LDASAVSLFD DHLGEDYYGS

310 320 330 340 350 360
EIYTLKEGLS SVPQGLLQAF LDAADSNEFY PNSHLKALRR KTNGQYVLYF EPTTSKDGQT

370 380 390 400 410 420
TINYLEPLQV VCAQRVILAM PVYALNQLDW NQLRNDRATQ AYAAVRPIPA SKVFMTFDQP

430 440 450 460 470 480
WWLENERKSW VTKSDALFSQ MYDWQKSEAS GDYILIASYA DGLKAQYLRE LKNQGEDIPG

490 500 510 520 530 540
SDPGYNQVTE PLKDTILDHL TEAYGVERDS IPEPVTAASQ FWTDYPFGCG WITWRAGFHF

550 560 570 580
DDVISTMRRP SLKDEVYVVG ADYSWGLISS WIEGALETSE NVINDYFL

In one embodiment, the fusion protein is zHER2:342(K4R/K7A/K27R/K49R/K50A/K58A)-APIT.
zHER2:342(K4R/K7A/K27R/K49R/K50A/K58A)-APIT comprises or consists of the amino acid sequence of SEQ ID NO. 5:

SEQ ID NO 5:                amino acid number: 589, molecular weight: 66036.77 g/mol

1         10         20         30         40         50         60
MVDNRFNAEMR NAYWEIALLP NLNNQQRRAF IRSLYDDPSQ SANLLAEARA LNDAQAPAGG

        70         80         90        100        110        120
GSGGGDGGGS MDGVSRNRRQ CNREVCGSTY DVAVVGAGPG GANSAYMLRD SGLDIAVFEY

       130        140        150        160        170        180
SDRVGGRLFT YQLPNTPDVN LEIGGMRFIE GAMHRLWRVI SELGLTPKVF KEGFGKEGRQ

       190        200        210        220        230        240
RFYLRGQSLT KKQVKSGDVP YDLSPEEKEN QGNLVEYYLE KLTGLQLNGE PLKREVALKL

       250        260        270        280        290        300
TVPDGRFLYD LSFDEAMDLV ASPEGKEFTR DTHVFTGEVT LDASAVSLFD DHLGEDYYGS

       310        320        330        340        350        360
EIYTLKEGLS SVPQGLLQAF LDAADSNEFY PNSHLKALRR KTNGQYVLYF EPTTSKDGQT

       370        380        390        400        410        420
TINYLEPLQV VCAQRVILAM PVYALNQLDW NQLRNDRATQ AYAAVRPIPA SKVFMTFDQP

       430        440        450        460        470        480
WWLENERKSW VTKSDALFSQ MYDWQKSEAS GDYILIASYA DGLKAQYLRE LKNQGEDIPG

490 500 510 520 530 540
SDPGYNQVTE PLKDTILDHL TEAYGVERDS IPEPVTAASQ FWTDYPFGCG WITWRAGFHF

550 560 570 580
DDVISTMRRP SLKDEVYVVG ADYSWGLISS WIEGALETSE NVINDYFL

The fusion protein zHER2:342(K4R/K7A/K27R/K49R/K50A/K58A)-APIT is highly suitable to be further modified, such as by PEGylation.

In a preferred embodiment, the active pharmaceutical ingredient (1) is modified, such as to increase its circulation time, enhance its serum half-life time, benefit the stability and/or increase enzyme activity of e.g. APIT.

Thereby, the targeting sequence can be modified and/or the protein (such as APIT) can comprise the modification.

A preferred embodiment for said modification is PEGylation, preferably with PEG sizes of about 1 to 40 kDa, more preferably up to 5 kDa.

Further suitable modification(s) are modification with poly(glycerols), poly(oxazolines), poly(amino acids), poly(acrylamides), poly(vinylpyrrolidones), and poly(zwitterions) and other modifications which, for example, benefit the stability and/or serum half-life time.

In one embodiment, the active pharmaceutical ingredient (1) is attached to a nanoparticle or the active pharmaceutical ingredient (1) is packed into/comprised within nanoparticles, such as lipid nanoparticles, liposomes or targeted nanoparticles.

### Active pharmaceutical ingredient (2) - prodrug

The active pharmaceutical ingredient (2) of the kit of the present invention is a prodrug or proreporter comprising a compound of formula I.

A "prodrug" as used herein refers to a compound which comprises a drug which is inactive as long as it is covalently attached to the compound but is active after its release. The drug will have its effect or activity after release.

A "proreporter" as used herein refers to a compound which comprises a reporter or imaging sensor which is inactive as long as it is covalently attached to the compound but is active after its release. When the reporter is, for example, a fluorescence dye, it will emit specific fluorescence only after release.

The compound of formula I or formula II 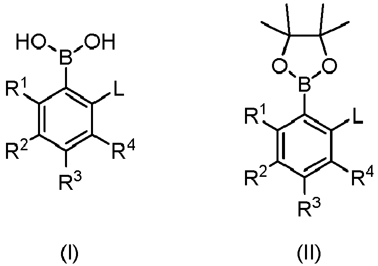 is a compound wherein
**R¹** to **R⁴** are each independently selected from H, O**R⁵**, NH**R⁵**, NHC(O)**R⁵**, S**R⁵**, halogen, C₁-C₆ alkyl, C₁-C₆ alkoxy, aryl, and C₁-C₂₀ alkyloxycarbonyl,
**R⁵** is selected from H, C₁-C₆ alkyl, C₁-C₆ alkoxy, and C₁-C₆ alkyl or C₁-C₆ alkoxy substituted with a functional group for attachment of a polymer, peptide or protein,
**L** is selected from -(CH₂)₂-C(O)O-**X** and -CH=CH-C(O)O-**X**, and
**X** is a drug or a reporter,
or a pharmaceutically acceptable salt thereof.

In a preferred embodiment, **X** is a drug.

Preferably, the drug is an anti-cancer drug, an anti-viral drug and/or an antibiotic.

Examples for anti-cancer drugs are camptothecin, doxorubicin, gemcitabine, and cytarabine. Examples for anti-viral drugs are 3'-hydroxyl nucleosides, ribavirin, ganciclovir, vidarabine, acyclovir, penciclovir, and zalcitabine.

Examples for antibiotics are aminoglycosides, streptomycin, chloramphenicol, tetracycline, macrolides, and geldanamycin.

In a preferred embodiment, **X** is a reporter or an imaging sensor.

Preferably, the reporter or imaging sensor is a fluorescence dye, a mass reporter/sensor or a chemoluminescence dye.

Examples for fluorescence dyes are near infrared (NIR) dyes, such as resorufin or 2-[2-(2,3-Dihydro-6-hydroxy-1*H*-xanthen-3-yl)ethenyl]-1-ethyl-3,3-dimethyl-3*H*-indolium. Examples for mass reporters/sensors are peptides and small molecules, which are isotopic labelled.

In one embodiment, where X is a drug, the compound of the present invention is a compound of formula Ia, Ib, IIa or IIb:

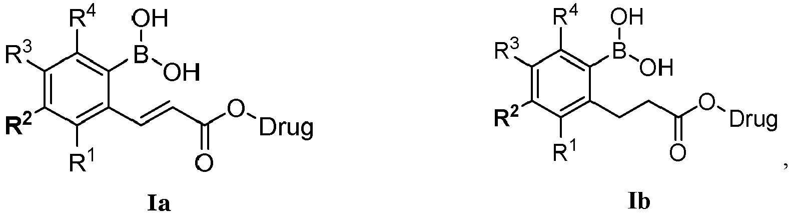

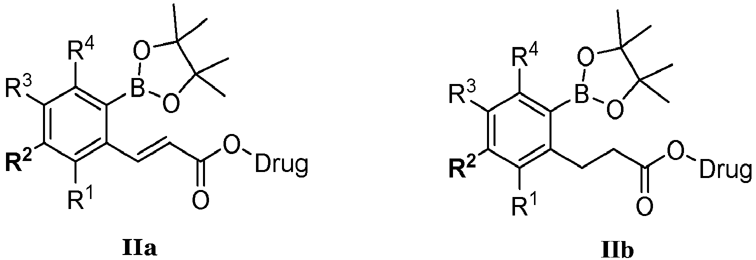

In embodiments of the active pharmaceutical ingredient (2) where X is a drug and said drug is camptothecin and the compounds are a compound of formula IIa or lib:

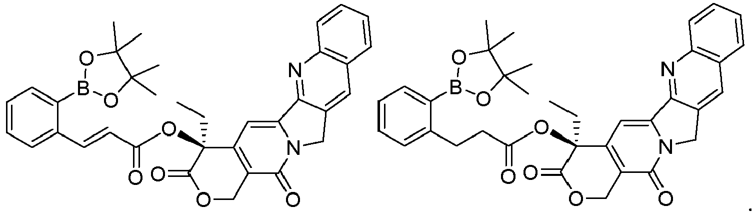

In a preferred embodiment,
**R¹**, **R³** and **R⁴** are each H, and
**R²** is selected from O**R⁵**, NH**R⁵**, NHC(O)**R⁵**, S**R⁵**, halogen, C₁-C₆ alkyl, C₁-C₆ alkoxy (such as OMe), aryl, and C₁-C₂₀ alkyloxycarbonyl.

In one embodiment,
**R¹**, **R³** and **R⁴** are each H,
**R²** is O**R⁵**, NH**R⁵**, NHC(O)**R⁵** or S**R⁵**, and
**R⁵** is selected from H, C₁-C₆ alkyl, C₁-C₆ alkoxy, and C₁-C₆ alkyl or C₁-C₆ alkoxy substituted with a reactive group for attachment of a polymer, peptide or protein.

Said reactive group for attachment of a polymer, peptide or protein is preferably selected from SH, maleimide, methacrylate, furan, an ene-compound, and a click substituent.

Examples for ene-compounds are alkenes, such as C₁-C₆ alkene.

Examples for click substituents are azide, alkyne, substituents comprising dibenzocyclooctyne (DBCO) group, substituents comprising trans-cycloctene (TCO) group, tetrazine.

For example, compounds where R² is NHR⁵ and R⁵ is H:

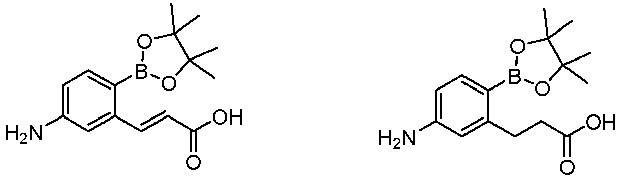

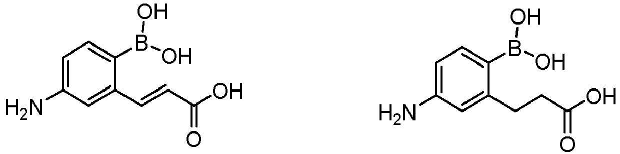

In one embodiment, the active pharmaceutical ingredient (2) comprises one or more further moieties.

Said one or more further moieties can serve in improving bioavailability.

A preferred further moiety for improving bioavailability is a polymer.

Said polymer can be covalently attached to the compound of formula I or II at any of **R¹ to R⁴**, preferably position **R²** wherein **R²** is preferably O**R⁵** or NHR⁵ or S**R⁵**, and **R⁵** is H.

An example is PEGylation, preferably with PEG sizes of about 1 to 40 kDa, more preferably up to 5 kDa.

Further examples are poly(glycerols), poly(oxazolines), poly(amino acids), poly(acrylamides), poly(vinylpyrrolidones), and poly(zwitterions).

In one embodiment, said further moiety is polyethylene glycol (PEG) which is covalently attached to the compound of formula I or II at any of **R¹** to **R⁴**, preferably position **R²** wherein **R²** is more preferably O**R⁵** or NH**R⁵** or and **R⁵** is H.

Said one or more further moieties can serve in improving selectivity.

Examples are peptides and proteins, such as tumor markers.

In one embodiment, said one further moiety is a peptide or protein which is covalently attached to the compound of formula I or II at any of **R¹** to **R⁴**, preferably position **R²** wherein **R²** is preferably O**R⁵**, NH**R⁵** or NHC(O) **R⁵** and **R⁵** is selected from C₁-C₆ alkyl or C₁-C₆ alkoxy substituted with a reactive group for attachment of a peptide or protein.

Said reactive group for attachment of a peptide or protein is preferably selected from SH, maleimide, methacrylate, furan, an ene-compound, and a click substituent, as discussed above.

Said reactive group is, for example, maleimide.

An example of a respective compound is the following compound: 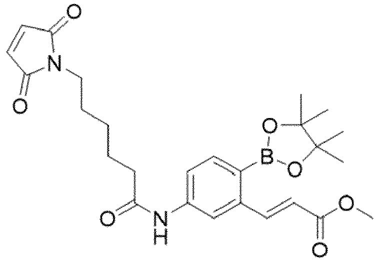 In this compound R² is NHC(O)R⁵ and R⁵ is pentyl (C₅ alkyl) which is substituted with a reactive group, wherein said reactive group is maleimide.

The significantly increased "ROS level" (for example, the amount of hydrogen peroxide (H₂O₂)) at the site of action, which is caused by the active pharmaceutical ingredient (1), is used to release the drug or reporter from the active pharmaceutical ingredient (2) at the site of action.

In one embodiment, the active pharmaceutical ingredient (1) is provided for administration before administration of the active pharmaceutical ingredient (2).

In one embodiment, the active pharmaceutical ingredient (1) and/or the active pharmaceutical ingredient (2) is provided for oral, intravenous and/or intratumoral administration.

In one embodiment, the active pharmaceutical ingredient (1) is provided for intravenous or intratumoral administration; and the active pharmaceutical ingredient (2) is provided for oral, intravenous and/or intratumoral administration.

In a preferred embodiment, the active pharmaceutical ingredient (1) is provided for intravenous administration; and the active pharmaceutical ingredient (2) is provided for oral administration, such as a tablet.

### Medical uses of the kit

As outlined above, the present invention provides the kit of the present invention for use in the diagnosis, prevention and/or treatment of proliferative diseases, viral infections and/or non-viral infections.

The "treatment" as used herein also comprises the alleviation of the treated disease.

In one embodiment, where the kit of the present invention is for use in the diagnosis of proliferative diseases, viral infections and/or non-viral infections, the active pharmaceutical ingredient (2) is preferably a proreporter, i.e. a compound of formula I wherein X is a reporter.

In one embodiment, where the kit of the present invention is for use in the prevention and/or treatment of proliferative diseases, viral infections and/or non-viral infections, the active pharmaceutical ingredient (2) is preferably a prodrug, i.e. a compound of formula I wherein X is a drug.

The proliferative disease is preferably cancer, more preferably a cancer with solid tumor(s).

The cancer is preferably selected from, but not limited to, lung cancer, multi drug resistant lung cancer, head and neck cancer, breast cancer, prostate cancer, colon cancer, cervix cancer, uterus cancer, larynx cancer, gastric cancer, liver cancer, Ewing's sarcoma, acute lymphoid leukemia, acute and chronic myeloid leukemia, apoptosis resistant leukemia, pancreas cancer, kidney cancer, gliomas, melanomas, chronic lymphoid leukemia, and lymphoma.

In one embodiment, the cancer is a HER2-positive cancer.

Examples for a HER2-positive cancer are breast cancer and gastric cancer.

In one embodiment, the cancer is a cancer with metabolic auxotrophy on the amino acids L-lysine and/or L-arginine, such as hepatocellular carcinoma, malignant melanoma, malignant pleural mesothelioma, and prostate and kidney cancers.

The viral infection is preferably an infection with human immunodeficiency virus (HIV), hepatitis B virus (HBV) or/and hepatitis C virus (HCV).

The non-viral infection is preferably a microbial infection.

In a preferred embodiment, the active pharmaceutical ingredient (1) is provided for administration before administration of the active pharmaceutical ingredient (2).

In one embodiment, the active pharmaceutical ingredient (1) is administered first, in order to unfold its effect on the disease tissue (which comprises degradation of L-lysine, L-arginine and, above all, the production of H₂O₂). In addition to its therapeutic effect, the H₂O₂ produced is to be used as a differentiating release stimulus of the drug from the active pharmaceutical ingredient (2).

In one embodiment, the active pharmaceutical ingredient (1) and/or the active pharmaceutical ingredient (2) is provided for oral, intravenous and/or intratumoral administration.

In one embodiment, the active pharmaceutical ingredient (1) is provided for intravenous or intratumoral administration; and the active pharmaceutical ingredient (2) is provided for oral, intravenous and/or intratumoral administration.

In a preferred embodiment, the active pharmaceutical ingredient (1) is provided for intravenous administration; and the active pharmaceutical ingredient (2) is provided for oral administration, such as a tablet.

In one embodiment, after the administration of the active pharmaceutical ingredient (2) an irradiation is carried out.

In said embodiment, the prodrug comprises the compound of formula I, wherein L is - CH=CH-C(O)O-X, i.e. comprises a double bond in the linker between the phenylboronic acid part and X. Due to the irradiation, such as with light having a wavelength of about 400 nm, there occurs an cis/trans isomerization unfolding the internal cyclization of the cis structure of the phenylboronic acid part to coumarin triggered by ROS and at the same time the X, such as a drug, is released.

Coumarin or 2H-chromen-2-one or 2H-1-Benzopyran-2-one:

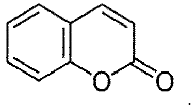

Coumarin derivatives can be fluorescent, depending on the substituents R¹ to R⁴ allowing a detection of the drug release.

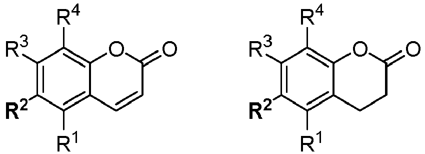

### Method of diagnosis and/or treatment

As outlined above, the present invention provides a method for the diagnosis, prevention and/or treatment of a proliferative disease, a viral infection and/or a non-viral infection.

Said method comprises the administration of therapeutically effective amount of the kit of the present invention to a subject in need thereof.

In one embodiment, where the method of the present invention is for the diagnosis of proliferative diseases, viral infections and/or non-viral infections, the active pharmaceutical ingredient (2) is preferably a proreporter, i.e. a compound of formula I wherein X is a reporter.

In one embodiment, where the method of the present invention is for the prevention and/or treatment of proliferative diseases, viral infections and/or non-viral infections, the active pharmaceutical ingredient (2) is preferably a prodrug, i.e. a compound of formula I wherein X is a drug.

In one embodiment, the method comprises the administration of a therapeutically effective amount of the active pharmaceutical ingredient (1) of the kit of the present invention to a subject in need thereof, and the subsequent administration of a therapeutically effective amount of the active pharmaceutical ingredient (2) of the kit of the present invention to said subj ect.

A "therapeutically effective amount" of a kit of the present invention, or of the active pharmaceutical ingredient (1) and/or (2), refers to the amount which has to be administered to a subject in need thereof in order to achieve a desired therapeutic result or outcome. The skilled artisan will be able to determine said therapeutically effective amount and the suitable administration regimen.

The "treatment" as used herein also comprises the alleviation of the treated disease.

The proliferative disease is preferably cancer, more preferably a cancer with solid tumor(s).

The cancer is preferably selected from, but not limited to, lung cancer, multi drug resistant lung cancer, head and neck cancer, breast cancer, prostate cancer, colon cancer, cervix cancer, uterus cancer, larynx cancer, gastric cancer, liver cancer, Ewing's sarcoma, acute lymphoid leukemia, acute and chronic myeloid leukemia, apoptosis resistant leukemia, pancreas cancer, kidney cancer, gliomas, melanomas, chronic lymphoid leukemia, and lymphoma.

In one embodiment, the cancer is a HER2-positive cancer.

Examples for a HER2-positive cancer are breast cancer and gastric cancer.

In one embodiment, the cancer is a cancer with metabolic auxotrophy on the amino acids L-lysine and/or L-arginine, such as hepatocellular carcinoma, malignant melanoma, malignant pleural mesothelioma, and prostate and kidney cancers.

The viral infection is preferably an infection with human immunodeficiency virus (HIV), hepatitis B virus (HBV) or/and hepatitis C virus (HCV).

The non-viral infection is preferably a microbial infection.

In a preferred embodiment, the active pharmaceutical ingredient (1) is administered before the active pharmaceutical ingredient (2).

Preferably, between the administration of the active pharmaceutical ingredient (1) and the administration of the active pharmaceutical ingredient (2) is a time period of at least one hour up to several days, preferably one hour to one day.

In one embodiment, the method comprises an irradiation after administration of the active pharmaceutical ingredient (2). As discussed above, in this embodiment the prodrug comprises the compound of formula I, wherein L is -CH=CH-C(O)O-X.

Preferably, the active pharmaceutical ingredient (1) and/or the active pharmaceutical ingredient (2) are administered via for oral, intravenous and/or intratumoral administration.

In one embodiment, the active pharmaceutical ingredient (1) is administered via intravenous or intratumoral administration; and the active pharmaceutical ingredient (2) is administered via oral, intravenous and/or intratumoral administration.

In a preferred embodiment, the active pharmaceutical ingredient (1) is administered via intravenous administration; and the active pharmaceutical ingredient (2) is administered via oral administration, such as via a tablet.

### Preferred embodiments

Herein, a novel treatment concept, which guarantees a selective increase of the ROS level in combination with targeted ROS-sensitive drug release ("prodrug" concept) by means of two modularly designed elements (pharmaceutically active ingredients) is described. See also Figure 1 C.

The pharmaceutically active ingredient (1) has the task of selectively generating oxidative stress and the associated increase in ROS molecules at the site of action.

Said significantly increased "ROS level" (for example, the amount of hydrogen peroxide (H₂O₂)) at the site of action is then used to release the drug from the pharmaceutically active ingredient (2) through the increased ROS.

### - Pharmaceutically active ingredient (1)

An antitumor enzyme (Aplysia punctata ink toxin (APIT)) was discovered in the defensive secretion of the marine sea hare Aplysia punctata. It is a weakly glycosylated FAD-binding L-amino acid oxidase (Figure 2 A) (Butzke *et al.,* 2005).

The antitumor effect of APIT is based on the selective degradation of essential L-amino acids (L-lysine and L-arginine). This redox reaction produces reactive byproducts (H₂O₂ and NH₄⁺), which are also detrimental to the tumor. Selective degradation of amino acids L-lysine and L-arginine is suitable for therapy of cancers with metabolic auxotrophy on these amino acids, such as hepatocellular carcinoma, malignant melanoma, malignant pleural mesothelioma, and prostate and kidney cancers (Butzke *et al.,* 2004). Since many tumor types lose the ability to produce arginine by converting citrulline in the urea cycle as they degenerate, the use of amino acid oxidases (for example, APIT) can be described as "starving" the cancer. In addition, reactive oxygen species (ROS) are produced during the enzyme reaction. It is known that tumors lose the ability to scavenge ROS (Perillo *et al.,* 2020). Therefore, a very high level of ROS leads to cell death and thus tumor death, which is an additional aspect of APIT's efficacy.

APIT showed a very short blood circulation time (~3 h) in initial experiments. Modifications using polyethylene glycol (PEG) showed a significant increase in blood circulation time and an enzymatic activity of more than 48 h. This system is already under development for cancer therapy under the name Oncarex^{®}, see also US patent 8,759,060 B2.

Further development of APIT is the subject of the combination therapy described here. To increase the specificity of APIT, it can be modularly provided with different targeting sequences. The first protein conjugate tested was a zHER2:342-APIT (see Figure 2 B and Figure 4). The zHER2:342 sequence promotes selective binding to HER2-overexpressing (HER: human epidermal growth factor receptor) cells (Dong *et al.,* 2016). This fusion protein was shown to bind to HER2-positive but not HER2-negative cells and to remain enzymatically active (see e.g. Figure 5).

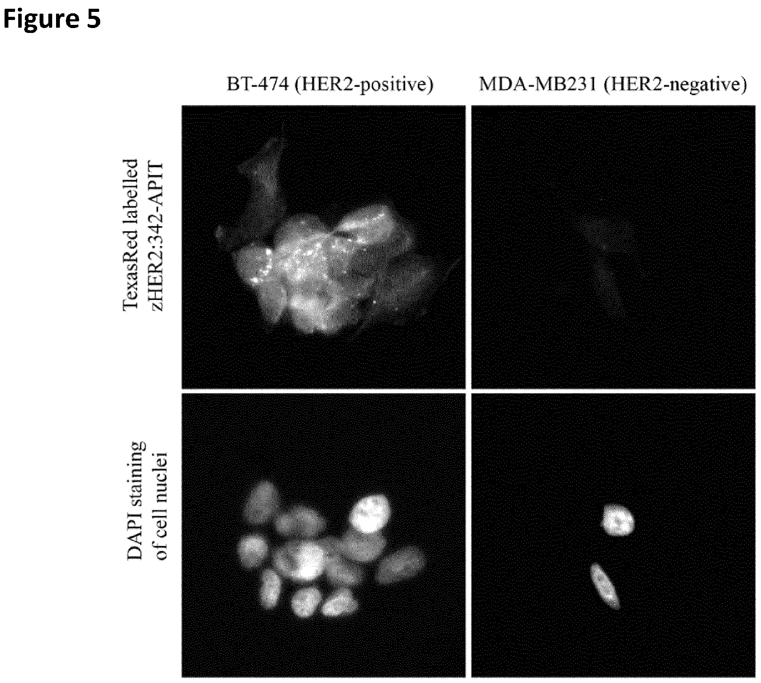

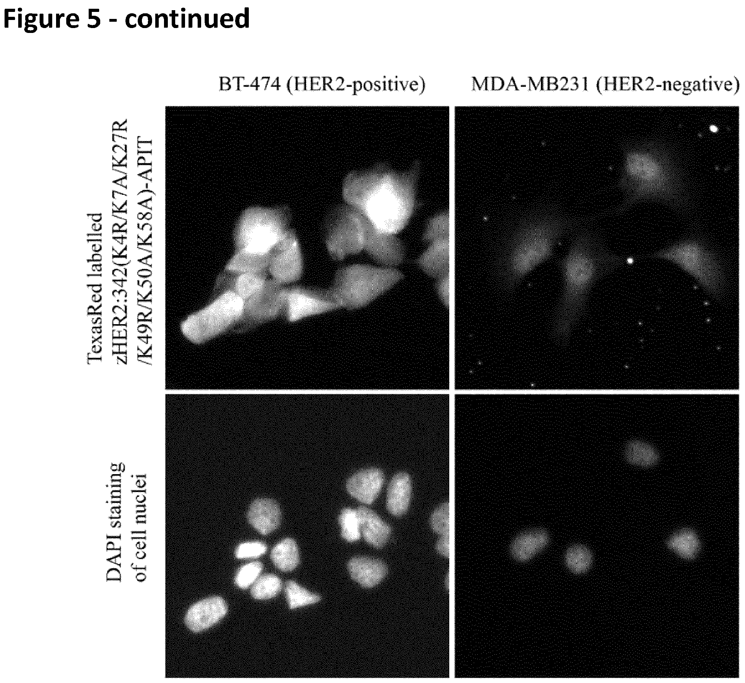

Furthermore, a previously undescribed lysine-deleted zHER2:342 mutant was synthesized as a fusion protein with APIT. The mutant was mutated to arginine in the case of functional properties of lysine such as surface charge or proximity to the HER2 binding site or to alanine in the case of structural properties of lysine. Starting from the native zHER2:342 sequence, the following mutations were made K4R/K7A/K27R/K49R/K50A/K58A. In silico stability studies show that the variant is more stable compared to the native zHER2:342 sequence. Again, the zHER2:342(K4R/K7A/K27R/K49R/K50A/K58A)-APIT fusion protein was shown to bind to HER2 positive but not HER2 negative cells and to be enzymatically active (see Figure 5).

There is another reason why this variant zHER2:342 is advantageous. As described above, the half-life of APIT can be extended by PEGylation. In this case, APIT can be inserted via the cheap and efficient reductive alkylation or via N-hydroxysuccinimidyl-based chemistry. Always, the nucleophilic lysine residues of APIT as well as its N-terminus are the starting point in these syntheses. The result is a randomized PEGylation of APIT (somewhere randomly PEGylated), i.e., a heterogeneous product with mixtures of different APIT:PEG stoichiometries. The conventional zHER2 also has lysine residues, and these too would be PEGylated in the fusion protein. Preliminary studies show that this causes the zHER2 to lose its targeting properties to the tumor. The new zHER2:342, which no longer contains lysine residues but still finds its way to the tumor, can circumvent this problem.

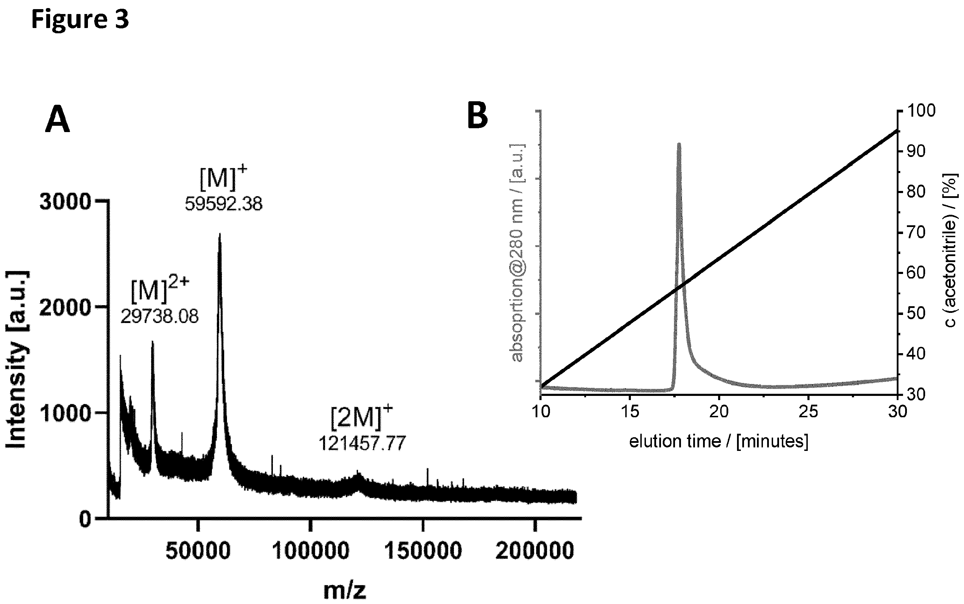

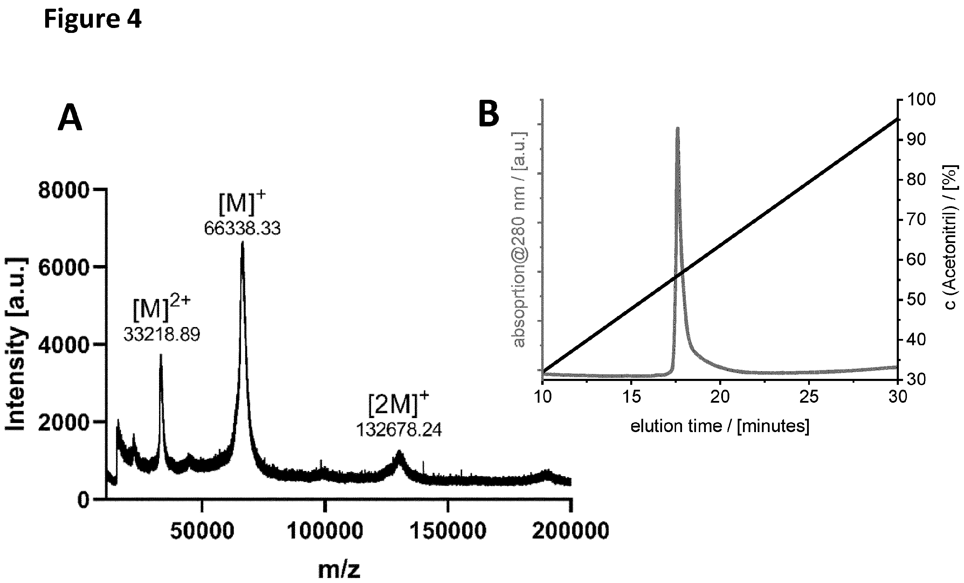

The proteins APIT, the fusion protein zHER2:342-APIT and zHER2:342(K4R/K7A/K27R/K49R/K50A/K58A)-APIT were successfully recombinantly produced and purified. The proteins were verified for identity and purity by mass spectrometry and high-performance liquid chromatography (HPLC) (see Figure 3 and 4). Subsequently, both proteins were compared with respect to their enzymatic activity, in particular the production of ROS (H₂O₂) via a spectrofluorimetric experimental setup. The activity of both species is very similar:
APIT: 80 U/mg,
zHER2:342-APIT: 70 U/mg,
zHER2:342(K4R/K7A/K27R/K49R/K50A/K58A)-APIT: 70 U/mg.

The ability of the zHER2:342-APIT and zHER2:342(K4R/K7A/K27R/K49R/K50A/K58A)-APIT fusion proteins to bind to HER2-positive cells was confirmed in a cell culture experiment (see Figure 5).

Important features of pharmaceutically active ingredient (1) are:
- Fusion proteins of APIT and a targeting sequence, exemplified by zHER2:342-APIT, can be produced recombinantly.
- Enzymatic activity and associated antitumor properties of the fusion protein are preserved.
- Targeting ability of zHER2:342 is preserved.

### - Pharmaceutically active ingredient (2)

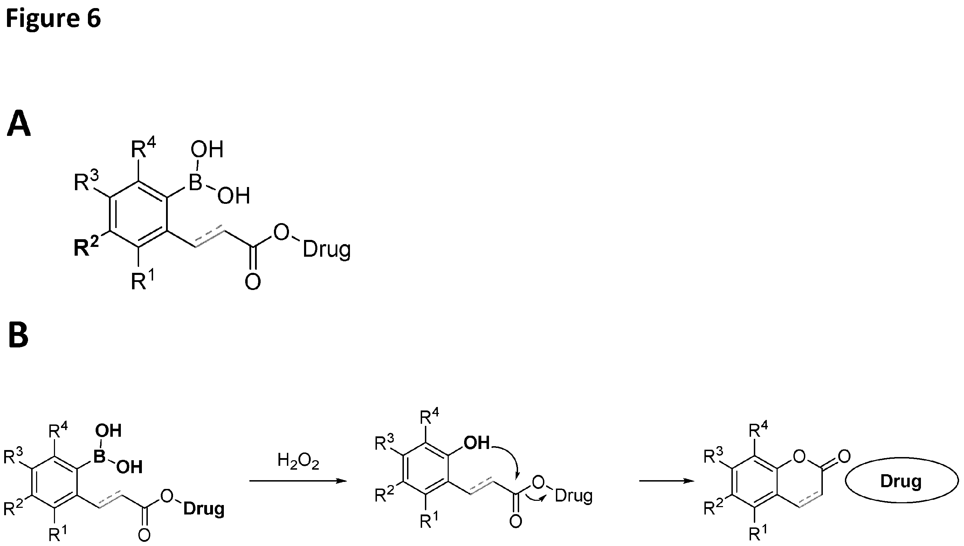

The pharmaceutically active ingredient (2) contains various phenylboronic acid derivatives (see e.g. Figure 6A).

The drug release cascade is started by the ROS (especially H₂O₂) driven conversion of the boronic acid to the alcohol (see Figure 6B). In the literature, the electronic pushing effect of the alcohol function is often used to release drugs in the para position (R² of formula I, II) (see Peiró Cadahía *et al.,* 2019; Wang *et al.,* 2016). In the pharmaceutically active ingredient (2) disclosed herein, drug release is achieved by intramolecular cyclization, namely attack of the alcohol function on the drug ester (Turner *et al.,* 1987; Greenwald *et al.,* 2000; Levine *et al.,* 2012; Okoh *et al.,* 2018; Walton and Dougherty, 2017). Thus, the R² position is still available for modifications.

See also Hankins *et al.,* 2020 and WO 2021/262998 A1, wherein aryl boronate and aryl diaminoboryl compounds are described, which can be used for detecting the presence of hydrogen peroxide and other ROS in water, a cell or tissue.

The required ring closure can optionally be controlled by light-induced *cis-trans* isomerization of the double bond (shown with the dotted line in Figure 4A and B) (Turner *et al.,* 1987; Gupta *et al.,* 2022; Gagey *et al.,* 2007). This dual controlled release mechanism ensures a controlled drug release and a coumarin derivative as a cyclization product is formed only at the site of action. As mentioned above, the aromatic provides additional opportunities for further functionalization (especially R²). On the one hand, this allows the spectroscopic properties of the resulting coumarin derivative to be modified (fluorescence for detection), but also other important modular modifications (protein, peptide and polymer conjugation).

In general, cyclization strategies and cis-trans control are well known. However, the combination with H₂O₂ selectivity in one construct is novel and very promising.

Important features of pharmaceutically active ingredient (2) are:
- Activity/toxicity of the coupled drug is greatly reduced.
- After release, efficacy is again fully guaranteed since no additional functionalities are present on the active ingredient at the end.
- Novel combination of ROS sensitivity, intramolecular cyclization reaction and cis-trans isomerization in one structure.
- Additional ring substitutions ensure the introduction of further structural motifs to improve bioavailability (polymer conjugation) and selectivity (tumor markers, proteins/peptides, zHER2:342).

### - Embodiments of the active pharmaceutical ingredient (2) with camptothecin

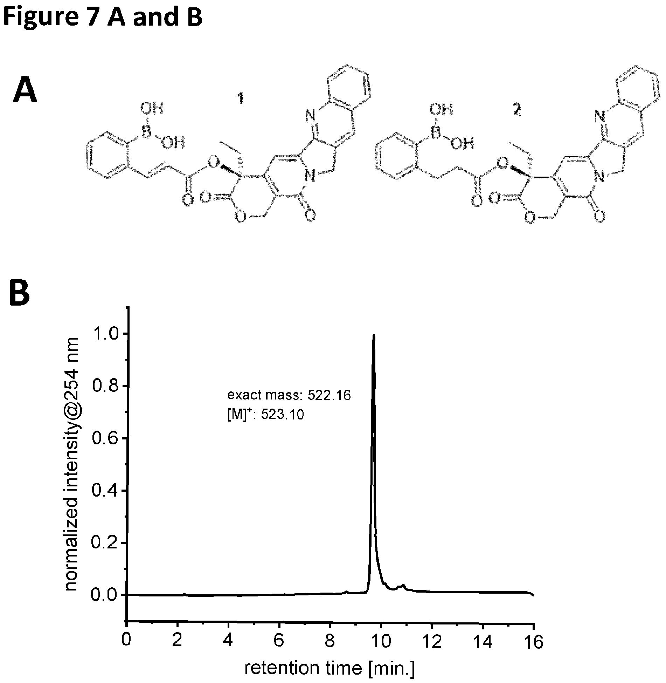

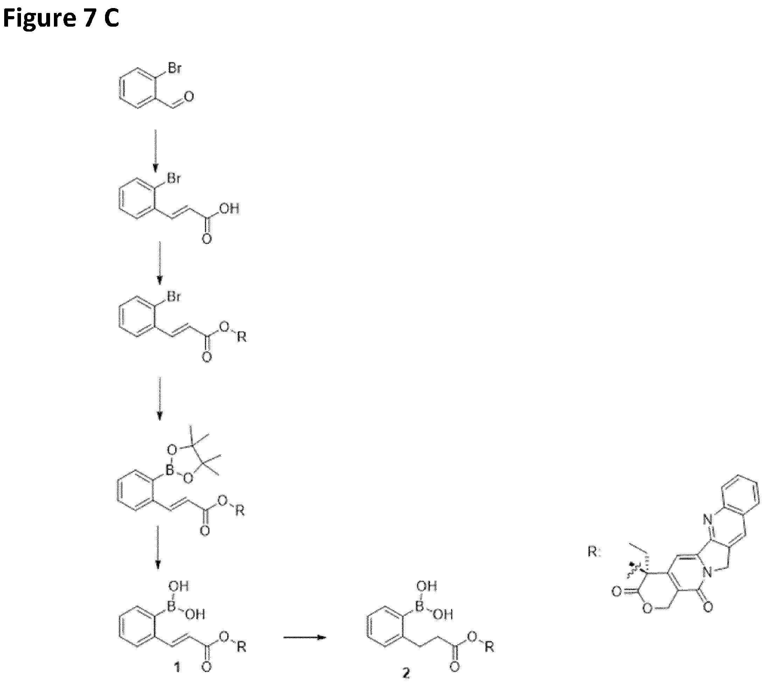

a) Compounds **1** and **2** (as shown in Figure 7A) were successfully synthesized at laboratory scale and analyzed by liquid chromatography with mass spectrometry coupling (LC-MS) (as shown in Figure 7B). The schematic synthesis pathway is shown in Figure 7C.

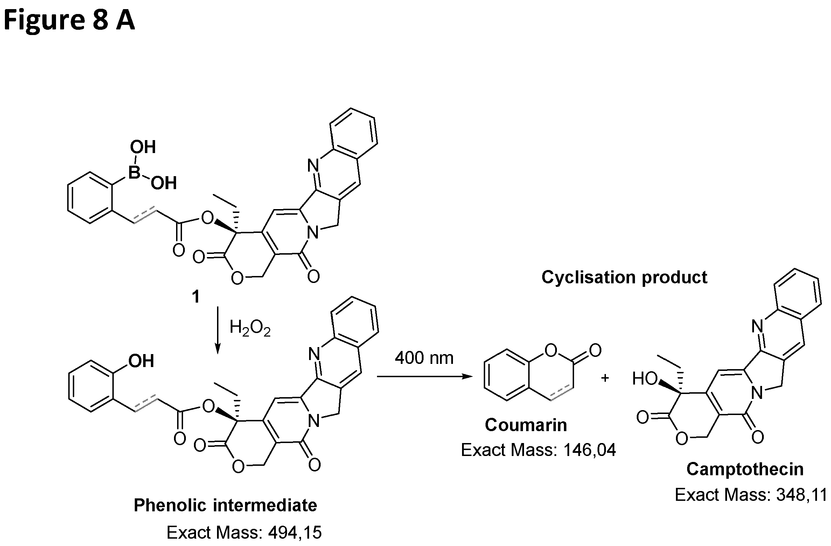

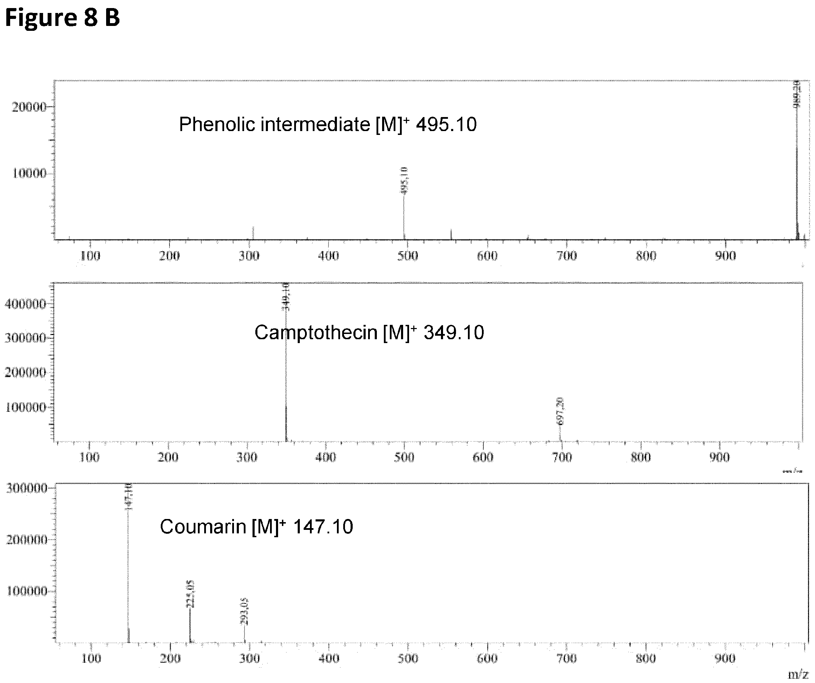

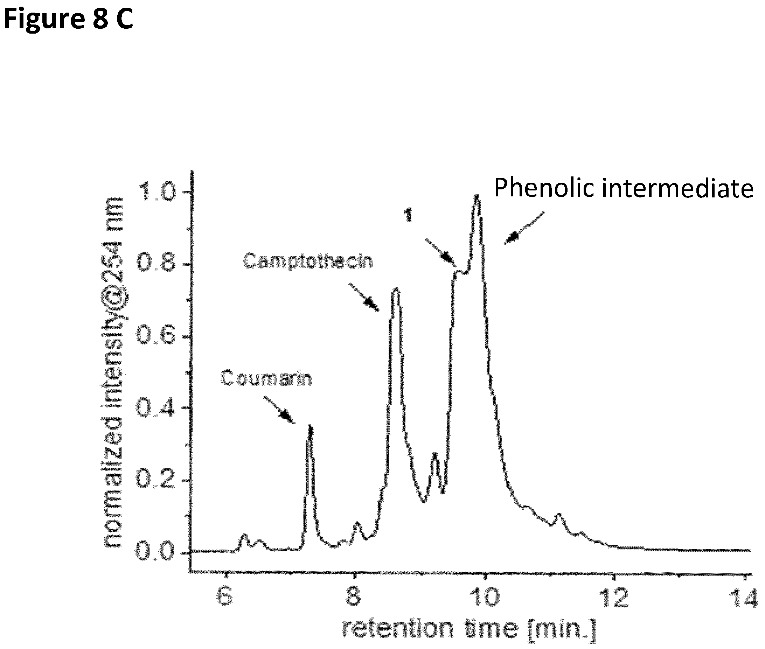

H₂O₂ sensitivity and, consequently, the drug-releasing cyclization mechanism by irradiation (λ = 400 nm) was confirmed by LC-MS (see Figure 8A, B and C).

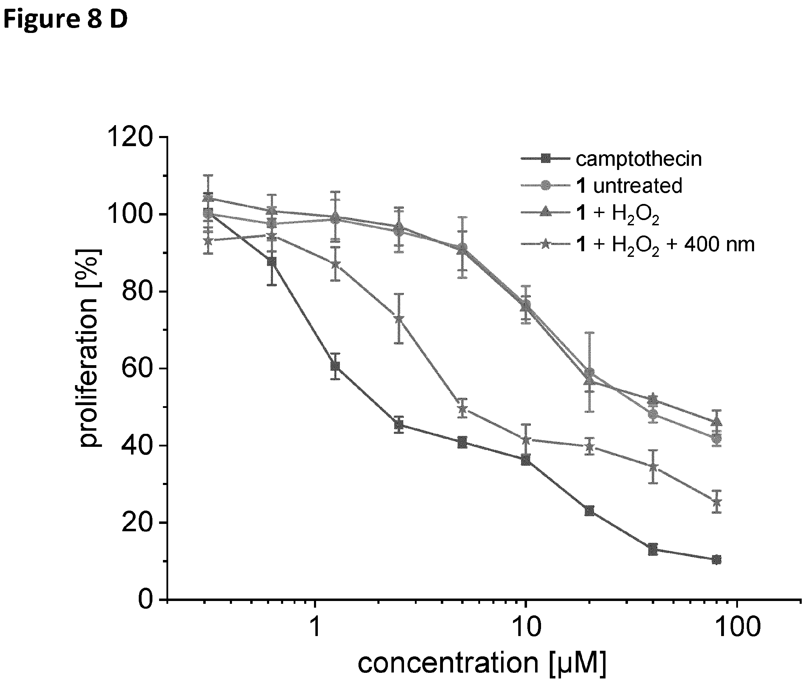

*In vitro* experiments demonstrate significantly reduced cytotoxicity of compound 1 compared to camptothecin (Figure 8D). Only after ROS treatment in combination with irradiation is increased cytotoxicity again observed due to the release of camptothecin.

b) As discussed herein, additional functionalities are possible on the aromatic ring of the central linker unit.

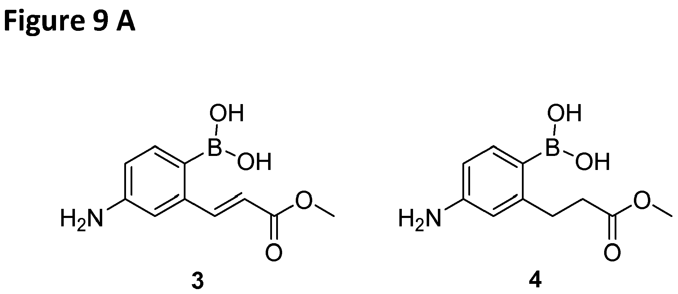

Compounds **3** and **4** which are compounds with an amine functionality at R² (as shown in Figure 9A) were successfully synthesized.

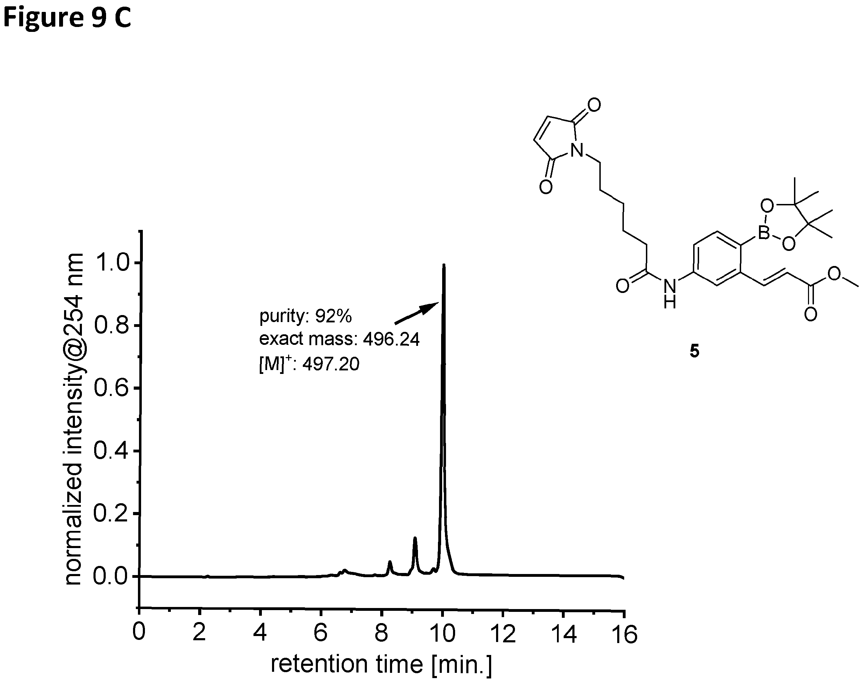

This amine functionality can be used for further reactions. An example of an acid-amine coupling is compound 5, as shown in Figure 9C, where a maleimide functionality was introduced. Said maleimide functionality is often used in protein and polymer conjugation. This functionality can also be used to change the spectroscopic properties of the resulting coumarin derivative for analytical purposes.

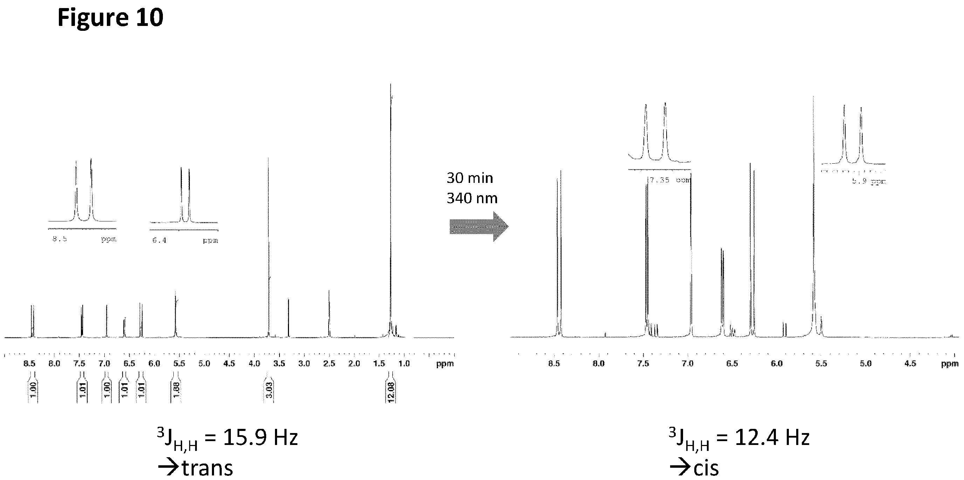

c) Another aspect of the cyclization mechanism is the cis-trans conformation of the double formation as for example in compounds 1, 3 and 5. The cis-trans conformation is controlled with light of specific wavelength, as shown for compound 3 in Figure 10.

The following examples and drawings illustrate the present invention without, however, limiting the same thereto.

### BRIEF DESCRIPTION OF THE DRAWINGS

**Figure 1****.** *Schematic representation of the principles of action.*
   Comparison of ROS levels in healthy tissue (blue bars) and diseased tissue (orange bars).
   A) Selective modulation of ROS levels in diseased tissue
   B) Natural ROS gradient as a release mechanism for ROS-sensitive "prodrugs".
   C) Combination of A and B to increase selectivity and efficacy.
**Figure 2****.** *Aplysia punctata ink toxin (APIT).*
   A) Mechanism of APIT catalyzed enzyme reaction.
   B) Schematic representation of the zHER2:342-APIT fusion protein. The zHER2:342 affinity domain is shown as a helical structure.
**Figure 3****.** *Recombinant APIT.*
   A) Analysis of the purified APIT by MALDI mass spectrometry (MALDI: matrix-assisted laser desorption ionization).
   B) HPLC elution of the protein APIT.
**Figure 4****.** *Fusion protein zHER2:342-APIT.*
   A) Analysis of purified zHER2:342-APIT by MALDI mass spectrometry.
   B) HPLC elution of the protein APIT.
**Figure 5****.** *Selective binding of the fusion proteins to HER2.*
   Shown are results of cell culture experiments to confirm the selective HER2 binding of the fusion proteins. The fusion proteins zHER2:342-APIT and zHER2:342(K4R/K7A/K27R/K49R/K50A/K58A)-APIT were labeled with fluorescent dye and probed for HER2 binding on fixed cells. Cell nuclei were stained with Hoechst33342. Left panels: HER2-positive cells (BT474 cell line). Here, the fluorescent dye labeled fusion proteins bind to the HER2 receptor located on the cell membrane.
   Right panels: HER2-negative cells (MDA-MB-231). The labeled fusion proteins do not bind specifically to the cell membrane (negative control).
**Figure 6****.** *Structure of component (2), the prodrug, and mechanism of drug release.*
   A) General structural formula of phenylboronic acid derivatives with coupled drug. Compounds with and without double bond (the respective double bond is shown with an interrupted line) are used.
   B) Drug release. In the first step, a conversion of boric acid to phenol is carried out by means of H₂O₂, followed by drug release by means of intramolecular cyclization reaction.
**Figure 7****.** *Embodiments of the active pharmaceutical ingredient (2) with camptothecin.*
   A) Structures of compounds 1 and 2.
   B) LC-MS analysis of compound 1.
   C) Example of a synthesis route of compounds 1 and 2.
**Figure 8****.** *Analysis of the drug release mechanism of one embodiment of the active pharmaceutical ingredient (2) with camptothecin and their activity on NIH3T3 fibroblasts.*
   A) Schematic of the reactions taking place: at first, a phenolic intermediate is formed under the influence of H₂O₂, and then due to irradiation at 400 nm camptothecin is released and a cyclization to coumarin occurs.
   B) Mass spectrometric analysis of the phenolic intermediate and the products camptothecin and coumarin.
   C) LC elution belonging to B).
   D) WST-1 assay on NIH3T3 fibroblasts. Compound **1** (untreated, circles) compared with camptothecin (control, squares) and different treatments (H₂O₂, molar ratio 2:1 (H₂O₂: compound 1), with (stars) and without irradiation (triangles)).

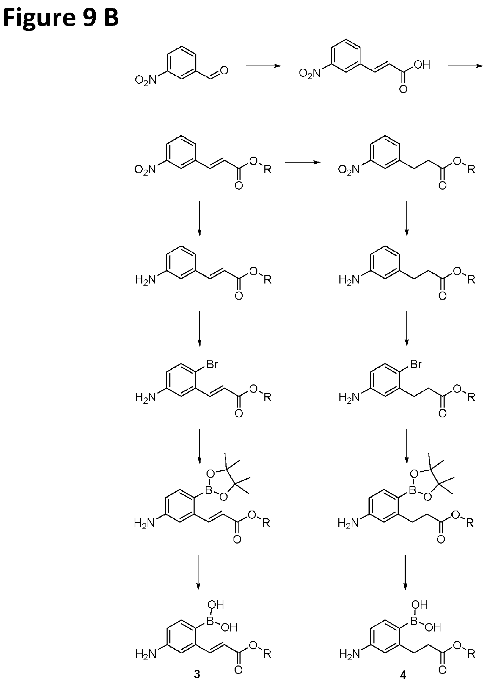

**Figure 9****.** *Embodiments of the active pharmaceutical ingredient (2) with a functionality at position R2.*
   A) Structures of compounds 3 and 4.
   B) Example of a synthesis route of compounds 3 and 4.
   C) Structure of compound 5, where a maleimide functionality was introduced, and LC-MS analysis of compound 5.
**Figure 10****.** *Light-induced cis-trans isomerization.*
   of compound 3, as an example.

### EXAMPLES

### EXAMPLE 1 Recombinant APIT and fusion proteins and their selective binding to target tumor cells.

### 1.1 Recombinant expression of APIT/ zHEr2APIT and variants

Competent *E. coli* HMS174 were transformed with the pET26b-based plasmid with inserted APIT/ zHER2APIT encoding gene between *Nde*I and *Eco*RI restriction sites. Expression was controlled by T7 promoter and induced by 1 mM IPTG (Sigma Aldrich, Steinheim, Germany) at an OD₆₀₀ of 0.8-1.0. After 4h cells were harvested by centrifugation at 5000 g, 4°C. Expression resulted in inclusion bodies. Pelleted cells were resuspended in 50 mm Tris pH 8.0. Inclusion bodies were isolated in several sonification steps using a Bandelin Sonoplus ultrasonic device (Berlin, Germany).

### 1.2. Refolding

0.5 g of purified inclusion bodies were solubilized in 100 mL 6 m Urea, 50 mm Tris, 200 mm NaCl, 100 mm DTT at pH 8.0 overnight at 4°C. Solubilized inclusion bodies were centrifuged at 5000 g, 4°C for 30 min and filtrated using a 0.2 µm PS-filter. The protein was diluted in refolding buffer 1 M Tris, 1 M NaCl, 1 mM EDTA, 25.6 µM FAD, 20 µM Cysteine, 0.5% PEG6000 pH 8.6 and stirred at 4°C for 7 days.

### 1.3. Purification of APIT

The subsequent purification of refolded protein was performed in two steps using an anion exchange chromatography (HiTrap^{™} Q FF) and a size exclusion chromatography (HiLoad^{™} 16/600 Superdex^{™} 200 pg) from Cytiva (Uppsala, Sweden).

Elution was performed with buffer A(50 mm Tris, pH 8.0) and buffer B (50 mm Tris, 1 m NaCl, pH 8.0), starting with a washing step of 25% B in 10 CV, followed by an elution gradient of 25-60 in 10 CV. APIT was then loaded onto a HiLoad^{™} 16/600 Superdex 200 pg. Column was then run using PBS pH 7.4 at flowrate of 1 mL/min.

### 1.4. PEGylation strategy

APIT, zHER2APIT or variant was dialyzed in PBS pH 7.4 overnight at 4°C and concentration was adjusted to 1.1 mg/mL. 20 eq of PEG-NHS 5kDa (Celares GmbH, Berlin, Germany) was dissolved at a concentration of 408 mg/mL in PBS pH 7.4 and 1: 10 (v:v) added to APIT. The reaction was performed overnight at 4°C continuously shaking. Reaction was purified using an anion exchange chromatography (HiTrap^{™} Q HP, Cytiva, Uppsala, Sweden) with buffer A (50 mm Tris, pH 8.0) and buffer B (50 mm Tris, 1 m NaCl, pH 8.0). The reaction was diluted 1:3 in buffer A, a maximum of 10 mg of protein was loaded onto 1 mL of resin at a flowrate of 0.5 mL/min. Afterwards a washing step at 0% B for at least 10 CV and elution gradient from 0-100% B was performed. Elution peak was pooled and dialyzed against PBS pH 7.4, concentration was adjusted to 2 mg/mL PEGylated APIT was filtered using a 0.2 µm PS filter and stored light protected.

### 1.5. Degree of PEGylation

The degree of PEGylation was assessed by determination of the mass increase measured by MALDI mass spectra with an ultrafleXtreme mass spectrometer (Bruker Daltonics, Bremen, Germany) equipped with a 355 nm smartbeam-II^{™} laser.

20-50 µg of protein were desalted using Sep-Pak Vac C18 cartridges. The recommended manufacturers instruction was followed. Bound protein was eluted using 80% acetonitrile in ddH₂O+ 0.1% TFA and subsequently lyophilized at -100°C, 0.001 mbar.

For external calibration Bruker Protein II standard (Bruker Daltonics #8207234) was mixed with sinapinic acid solution (saturated in a 1:2 mixture (v/v) of acetonitrile and 0.1% TFA). Drops were measured on a stainless-steel target (MTP 384 target plate ground steel, Bruker Daltonics #8280784). The proteins were analyzed after double layer preparation. First a thin matrix layer of SA solution (saturated in ethanol) was applied onto the stainless-steel target, second the sample solution (4 mg/mL in a 1:1 mixture of acetonitrile and ddH2O) was mixed with sinapinic acid solution (saturated in a 1:2 mixture (v/v) of acetonitrile and 0.1% TFA) and dropped onto the first layer. Several thousand shots were accumulated for the spectrum.

### 1.6 Fluorescent labelling of APIT and zHER2-APIT

Labelling of 1 eq protein was performed with either 6 eq of AlexaFluor680^{™}-maleimide (NIR) or 10 eq of TexasRed-maleimide in PBS containing 50 mM TCEP pH 7.4 at a protein concentration of 1.0 mg/mL overnight. Reaction mixture was purified via HiTrap Q HP using 50 mM Tris pH 8.0 (A) and 50 mM Tris 1 M NaCl pH 8.0 (B) with following method: 30% B for 45 CV, 30-100% B in 30 CV The eluent was dialyzed in PBS. The degree of label (DOL) was determined according to the manufacturer's instructions using extinctions coefficient of 1.539 mLmg-lcm-1 for APIT.

### 1.7. Endotoxin reduction

Endotoxin amount was tested using the EndoZyme II-kit. Endotoxin amount in one injection of 100 µL should not exceed 3.6 EU. For endotoxin reduction an anion-exchange chromatography was used. All chemicals were purchased in at least American chemical society (ACS) grade. All equipment required was either endotoxin reduced by incubation in or flushing with 1 M NaOH for at least 6 h or baked at 200°C for at least 6 h. Protein was loaded at low conductivity and elution was performed accordingly using 50 mM Tris pH 8.0 (A) and 50 mM Tris 1 M NaCl pH 8.0 (B): 0% B for 15 CV, 0-100% B in 50 CV

### 1.8. Cell-based HER-2 binding assay

HER2 positive BT-474 (ACC 64), adherent cells were cultured in RPMI 1640 containing 20% (v/v) FBS, MEM non-essential amino acids, 1 mM Pyruvate, 10 µg/ml human insulin. HER2-negative MDA-MB-231 (ACC 732) adherent cells were cultured in Leibowitz' L15, 20% FBS. Cells were cultured from frozen stocks and passaged two times prior to the assay. Cells were incubated at 37°C, 5% CO2 humidified. At a confluency of 80% cell passaging was performed by trypsination using 0.25% (w/v) trypsin/ 0.2% (w/v) EDTA. Cells were seeded at 10 k cells/ well on sterilized degreased 4%-gelatin coated cover slides (12 nm) in 24-well plates and grown over night in the incubator. Cells were washed with PBS and fixated with freshly made 4%

(w/v) paraformaldehyde solution for 30 min. Afterwards, cells were washed with PBS and incubated with eighter 1:100 dilution of HER2 antibody or 10 µg/mL zHER2-APIT-TxR each in PBS pH 7.4 overnight at 4°C protected from light. Afterwards, fixated cells were washed with PBS, the secondary anti-rabbit-AlexaFluor488 was added to the HER2-staining for 1 h and washed again. Cell nuclei were stained for 10 min using Hoechst33342. Cells were washed again and mounted using Mowiol-488-solution. Fluorescence signal was imaged using a ZEISS motorized Axio Z1 observer fluorescence microscope. Images were analyzed and processed using Zeiss Zen Pro software.

### EXAMPLE 2 Synthesis of functionalized and H₂O₂-sensitive phenylboronic acid derivatives and H₂O₂-induced drug release.

### 2.1. Chemicals

All chemicals were purchased from Sigma Aldrich and used without further purification.

### 2.2. Synthesis of (E)-3-(2-(4,4,5,5 tetramethyl-1,3,2-dioxaborolan-2-yl)phenyl)acrylic acid

1 Equivalent of 2-bromobenzaldehyde and 1.3 equivalents of malonic acid were stirred in a mixture of piperdine and pyridine at 110 °C for 90 minutes. Water and HCl (37%) were added to the reaction mixture and the resulting precipitate was filtered off and washed with water. 1 Equivalent of the white crystalline solid, 0.05 equivalents of Pd(dppf)Cl₂, 1.5 equivalents of bis(pinacolato)diboron and 1.5 equivalents of potassium acetate in anhydrous 1,4-dioxane was heated to 80 °C by means of microwave irradiation and stirred for 5 hours. After addition of diethyl ether, the reaction mixture was filtered through a plug of cellite, washed with diethyl ether and concentrated in vacuo. The crude product was purified by column chromatography.

The features disclosed in the foregoing description, in the claims and/or in the accompanying drawings may, both separately and in any combination thereof, be material for realizing the invention in diverse forms thereof.

### REFERENCES

Butzke, D., et al., Hydrogen peroxide produced by Aplysia ink toxin kills tumor cells independent of apoptosis via peroxiredoxin I sensitive pathways. Cell Death & Differentiation, 2004. 11(6): p. 608-617.
Butzke, D., et al., Cloning and biochemical characterization of APIT, a new l-amino acid oxidase from Aplysia punctata. Toxicon, 2005. 46(5): p. 479-489.
Dong, D., et al., Human Serum Albumin and HER2-Binding Affibody Fusion Proteins for Targeted Delivery of Fatty Acid Modified Molecules and Therapy. Molecular Pharmaceutics, 2016. 13(10): p. 3370-3380.
Exner, N., et al., Mitochondrial dysfunction in Parkinson's disease: molecular mechanisms and pathophysiological consequences. TheEMBO Journal, 2012. 31(14): p. 3038-3062.
Gagey, N., et al., Two Photon Uncaging with Fluorescence Reporting: Evaluation of the o-Hydroxycinnamic Platform. Journal of the American Chemical Society, 2007. 129(32): p. 9986-9998.
Greenwald, R.B., et al., Drug Delivery Systems Based on Trimethyl Lock Lactonization: Poly(ethylene glycol) Prodrugs of Amino-Containing Compounds. Journal of Medicinal Chemistry, 2000. 43(3): p. 475-487.
Gupta, A., A. Gautam, and P.K. Sasmal, Photoactivatable o-Hydroxycinnamic Platforms for Bioimaging and Therapeutic Release. Journal of Medicinal Chemistry, 2022.
Hankins RA, Suarez SI, Kalk MA, Green NM, Harty MN, Lukesh III JC, An innovative hydrogen peroxide-sensing scaffold and insight towards its potential as an ros-activated persulfide donor. Angew. Chem. Int. Ed., 2020. DOI: 10.1002/anie.202010530
Johri, A. and M.F. Beal, Antioxidants in Huntington's disease. Biochimica et Biophysica Acta (BBA) - Molecular Basis of Disease, 2012. 1822(5): p. 664-674.
Levine, M.N. and R.T. Raines, Trimethyl lock: a trigger for molecular release in chemistry, biology, and pharmacology. Chemical Science, 2012. 3(8): p. 2412-2420.
Mattson, M.P., Pathways towards and away from Alzheimer's disease. Nature, 2004. 430(7000): p. 631-639.
Okoh, O.A. and P. Klahn, Trimethyl Lock: A Multifunctional Molecular Tool for Drug Delivery, Cellular Imaging, and Stimuli-Responsive Materials. ChemBioChem, 2018. 19(16): p. 1668-1694.
Peiró Cadahía, J., et al., Prodrug strategies for targeted therapy triggered by reactive oxygen species. MedChemComm, 2019. 10(9): p. 1531-1549.
Peng, X and Gandhi V. ROS-activated anticancer prodrugs: a new strategy for tumor-specific damage. Therapeutic delivery, 2012 3(7): 823-833.
Perillo, B., et al., ROS in cancer therapy: the bright side of the moon. Experimental & Molecular Medicine, 2020. 52(2): p. 192-203.
Reczek, C.R. and N.S. Chandel, The Two Faces of Reactive Oxygen Species in Cancer. Annual Review of Cancer Biology, 2017. 1(1): p. 79-98.
Sies, H. and D.P. Jones, Reactive oxygen species (ROS) as pleiotropic physiological signalling agents. Nature Reviews Molecular Cell Biology, 2020. 21(7): p. 363-383.
Turner, A.D., et al., Photochemical activation of acylated .alpha.-thrombin. Journal of the American Chemical Society, 1987. 109(4): p. 1274-1275.
Walton, D.P. and D.A. Dougherty, A General Strategy for Visible-Light Decaging Based on the Quinone Trimethyl Lock. Journal of the American Chemical Society, 2017. 139(13): p. 4655-4658.
Wang, J., et al., Leveraging H2O2 Levels for Biomedical Applications. Advanced Biosystems, 2017. 1(9): p. 1700084.

## Claims

1. A kit of active pharmaceutical ingredients comprising
(1) a fusion component comprising an agent, which increases the level of reactive oxygen species (ROS), and a targeting sequence, and
(2) a prodrug or proreporter comprising a compound of formula I or formula II 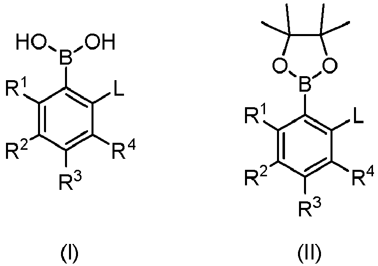 wherein
**R¹ to R⁴** are each independently selected from H, O**R⁵**, NH**R⁵**, NHC(O)**R⁵**, S**R⁵**, halogen, C₁-C₆ alkyl, C₁-C₆ alkoxy, aryl, and C₁-C₂₀ alkyloxycarbonyl,
**R⁵** is selected from H, C₁-C₆ alkyl, C₁-C₆ alkoxy, and C₁-C₆ alkyl or C₁-C₆ alkoxy substituted with a reactive group for attachment of a polymer, peptide or protein,
**L** is selected from -(CH₂)₂-C(O)O-**X** and -CH=CH-C(O)O-**X,** and
**X** is a drug or a reporter,
or a pharmaceutically acceptable salt thereof.

2. The kit of claim 1, wherein the fusion component is a fusion protein and the agent, which increases the level of reactive oxygen species (ROS) is a protein, preferably an L-amino acid oxidase, more preferably Aplysia punctata ink toxin (APIT), which comprises or consists of the amino acid sequence of SEQ ID NO. 1,
and/or wherein the targeting sequence of active pharmaceutical ingredient (1) targets cancer cells, viruses, bacterial cells or cells of other microorganisms.

3. The kit of claim 1 or 2, wherein the targeting sequence of active pharmaceutical ingredient (1) targets human epidermal growth factor receptor (HER2)-overexpressing cells, and is preferably
zHER2:342 comprising or consisting of the amino acid sequence of SEQ ID NO. 2, or
a zHER2:342 variant wherein the lysine residues are deleted or substituted, preferably with arginine and/or alanine, more preferably K4R/K7A/K27R/K49R/K50A/K58A zHER2:342 comprising or consisting of the amino acid sequence of SEQ ID NO. 3.

4. The kit of any one of claims 1 to 3, wherein the active pharmaceutical ingredient (1) comprises or consists of a fusion protein having the amino acid sequence of SEQ ID NO. 4 or 5.

5. The kit of any one of the preceding claims, wherein in the active pharmaceutical ingredient (2)
**R¹**, **R³** and **R⁴** are each H, and
**R²** is selected from O**R⁵**, NH**R⁵**, NHC(O) **R⁵**, S**R⁵**, halogen, C₁-C₆ alkyl, C₁-C₆ alkoxy, aryl and C₁-C₂₀ alkyloxycarbonyl.

6. The kit of claim 5, wherein **R²** is O**R⁵**, NH**R⁵**, NHC(O) **R⁵** or S**R⁵** and **R⁵** is selected from H, C₁-C₆ alkyl, C₁-C₆ alkoxy, and C₁-C₆ alkyl or C₁-C₆ alkoxy substituted with a reactive group for attachment of a polymer, peptide or protein,
wherein said reactive group is preferably selected from SH, maleimide, methacrylate, furan, an ene-compound, and a click substituent.

7. The kit of any one of the preceding claims, wherein **X** is
a drug which is selected from
anti-cancer drugs, such as camptothecin, doxorubicin, gemcitabine, and cytarabine,
anti-viral drugs, such as 3'-hydroxyl nucleosides, ribavirin, ganciclovir, vidarabine, acyclovir, penciclovir, and zalcitabine; and/or
antibiotics, such as are aminoglycosides, streptomycin, chloramphenicol, tetracycline, macrolides, and geldanamycin;
or
a reporter which is selected from
fluorescence dyes, such as near infrared (NIR) dyes, e.g. resorufin or 2-[2-(2,3-Dihydro-6-hydroxy-1*H*-xanthen-3-yl)ethenyl]-1-ethyl-3,3-dimethyl-3*H*-indolium
mass reporters, and
chemoluminescence dyes.

8. The kit of any one of the preceding claims, wherein the active pharmaceutical ingredient (2) comprises one or more further moieties,
wherein one further moiety is preferably a polymer, more preferably polyethylene glycol (PEG),
wherein said polymer is preferably attached to the compound of formula I or II at any of **R¹ to R⁴**, more preferably position **R²** wherein **R²** is more preferably O**R⁵**, NH**R⁵** or S**R⁵** and **R⁵** is H;
and/or
wherein one further moiety is a peptide or a protein,
wherein said peptide or protein is covalently attached to the compound of formula I or II at any of **R¹** to **R⁴**, preferably position **R²**, wherein **R²** is preferably O**R⁵**, NH**R⁵** or NHC(O) **R⁵** and **R⁵** is selected from C₁-C₆ alkyl or C₁-C₆ alkoxy substituted with a reactive group for attachment of a peptide or protein being selected from SH, maleimide, methacrylate, furan, an ene-compound, and a click substituent.

9. The kit of any one of the preceding claims, wherein the active pharmaceutical ingredient (1) is provided for administration before administration of the active pharmaceutical ingredient (2), and/or
wherein the active pharmaceutical ingredient (1) and/or active pharmaceutical ingredient (2) are provided for oral, intravenous and/or intratumoral administration.

10. The kit of any one of claims 1 to 9 for use in the diagnosis, prevention and/or treatment of proliferative diseases, viral infections and/or non-viral infections.

11. The kit for use according to claim 10, wherein the proliferative disease is cancer, preferably a cancer with solid tumor(s),
and/or wherein the viral infection is an infection with human immunodeficiency virus (HIV), hepatitis B virus (HBV) or/and hepatitis C virus (HCV),
and/or wherein the non-viral infection is a microbial infection.

12. The kit for use according to claim 10 or 11, wherein the active pharmaceutical ingredient (1) is provided for administration before the administration of active pharmaceutical ingredient (2), and/or
wherein the active pharmaceutical ingredient (1) and/or the active pharmaceutical ingredient (2) are administered via oral, intravenous and/or intratumoral administration.

13. The kit for use according to any one of claims 10 to 12, comprising an irradiation after administration of the active pharmaceutical ingredient (2),
preferably when **L** is -CH=CH-C(O)O-**X.**

14. A method for the diagnosis, prevention and/or treatment of a proliferative disease, a viral infection and/or a non-viral infection,
comprising the administration of therapeutically effective amounts of the kit of any one of claims 1 to 9 to a subject in need thereof.

15. The method of claim 14,
wherein the proliferative disease is cancer, preferably a cancer with solid tumor(s),
and/or wherein the viral infection is an infection with human immunodeficiency virus (HIV), hepatitis B virus (HBV) or/and hepatitis C virus (HCV),
and/or wherein the non-viral infection is a microbial infection.
and/or
wherein the active pharmaceutical ingredient (1) is administered before the active pharmaceutical ingredient (2),
and/or
wherein between the administration of active pharmaceutical ingredient (1) and active pharmaceutical ingredient (2) is a time period of at least one hour up to several days, preferably one hour to one day,
and/or
wherein the method comprises an irradiation after administration of the active pharmaceutical ingredient (2),
and/or
wherein the active pharmaceutical ingredient (1) and/or the active pharmaceutical ingredient (2) are administered via oral, intravenous and/or intratumoral administration.
